# EUROPÄISCHE PATENTSCHRIFT

(11) **EP 1 965 879 B1**
(45) Veröffentlichungstag und Bekanntmachung des Hinweises auf die Patenterteilung: **13.05.2009**
(21) Anmeldenummer: 06841344.2
(22) Anmeldetag: 13.12.2006
(51) Int. Cl.: B01D 9/02, C07C 29/70, C07C 29/78

(54) **VERFAHREN ZUR GEWINNUNG VON ALKOHOLATEN**
PROCESS FOR OBTAINING ALCOHOLATES
PROCEDE D' OBTENTION D'ALCOOLATES

(30) Priorität: 23.12.2005 DE 102005062654
(43) Veröffentlichungstag der Anmeldung: 10.09.2008
(73) Patentinhaber: BASF SE, 67056 Ludwigshafen (DE)
(72) Erfinder: ECK, Bernd, 68519 Viernheim (DE); RAULS, Matthias, 67061 Ludwigshafen (DE); FEISE, Hermann Josef, 67259 Kleinniedesheim (DE); LETZELTER, Thomas, 76855 Annweiler (DE); GUTH, Josef, 67251 Freinsheim (DE)
(86) Internationale Anmeldenummer: PCT/EP2006/069630
(87) Internationale Veröffentlichungsnummer: WO 2007/074061

(56) Entgegenhaltungen:
- WO-A-99/65849
- DE-A1- 10 158 353
- DE-A1- 19 920 594
- US-A- 3 587 704

## Beschreibung

Die vorliegende Erfindung betrifft ein Verfahren zur Gewinnung von Alkoholaten durch Kristallisation.

Alkoholate leiten sich von Alkoholen R-OH durch Ersatz des Wasserstoffatoms der Hydroxigruppe durch ein Metall M ab. Formal sind sie also Metallsalze R-OM der Alkohole R-OH. Alkoholate, ihre Herstellung und Verwendung sind wohlbekannt. Am weitesten verbreitet sind die Alkalialkoholate. Alkalialkoholate (systematischer Name: "Alkalialkanolate", auch "Alkalialkoxide" ist ein gebräuchliches Synonym) sind wohlbekannte Reagenzien in der organischen Chemie. Sie werden dort, wo starke Basen als Reaktionspartner benötigt werden, und als Katalysatoren bestimmter Reaktionen eingesetzt. In größeren Mengen hergestellt und verwendet werden praktisch nur aliphatische Alkalialkoholate des Lithiums, Natriums und Kaliums mit 1 bis 4 C-Atomen im Alkylrest des Alkohols, insbesondere Lithium-, Natrium- und Kalium- -methylat, - ethylat, -n-propylat, -iso-propylat, -n-butylat und -tert.-butylat. Verfahren zur Herstellung von Alkalialkoholaten sind seit langem bekannt. Einen allgemeinen Überblick über aliphatische Alkalialkoholate, ihre Herstellung und Verwendung gibt beispielsweise Ullmann's Encyclopedia of Industrial Chemistry, Sixth Edition, Band 2, WILEY-VCH Verlag GmbH & Co. KGaA, Weinheim 2003 (ISBN 3-527-30385-5), als Punkt 4. unter dem Stichwort "Alcohols, Aliphatic". Bekannte Verfahren zur Herstellung von Alkalialkoholaten sind die direkte Umsetzung von Alkalimetall mit Alkohol und die Umsetzung von Alkalihydroxid mit Alkohol unter destillativer Abtrennung des entstehenden Wassers, wie z. B. im deutschen Patent DE 519 443, oder in einer anderen Ausgestaltung in WO 01/42 178 A1, DE 10158353 oder in DE 19920594 beschrieben. Die Umsetzung von Alkalihydroxid mit Alkohol unter Abtrennung des Nebenprodukts Wasser ist vergleichsweise energieaufwendig, da das Nebenprodukt Wasser nur als geringer Anteil in einer großen abdestillierten und in die Reaktion zurückzuführenden Alkoholmenge ausgeschleppt werden kann. Bei manchen derartigen Verfahrensvarianten ist auch noch ein Lösungs- oder Schleppmittelkreislauf durch die Destillation notwendig, wie etwa bei den im deutschen Patent Nr. 505 474, in US 1 816 843 oder im französischen Patent Nr. 1 089 101 beschrieben Verfahren. Ein besonders aufwendiges Spezialverfahren zur Herstellung von Alkoholaten höherer Alkohole, die durch direkte Umsetzung nicht zugänglich sind, ist die Umsetzung von Alkalimethylat oder Alkaliethylat mit dem höheren Alkohol unter destillativer Abtrennung von Methanol oder Ethanol.

Die direkte Umsetzung eines Alkalimetalls mit einem Alkohol ist eine einfache und wirtschaftliche und daher die technisch am weitesten verbreitete Herstellweise für Alkalialkoholate. Die Reaktivität der Alkalimetalle steigt in der Reihenfolge Lithium, Natrium, Kalium, Rubidium und Cäsium, und die Reaktivität der Alkohole sinkt mit dem Molekulargewicht des Alkohols und dem Verzweigungsgrad des Alkylrests. Die Umsetzung wird oft mit einer Dispersion des Alkalimetalls in einem inerten Lösungsmittel oder besonders bequem mit Alkaliamalgam durchgeführt. Einen Überblick über die technische Herstellung von Alkalialkoholaten aus Alkaliamalgam und dazu verwendete Reaktionsapparate gibt z. B: R. B. MacMullin in: "By-Products of Amalgam-type Chlorine Cell", Chemical Engineering Progress Sept. 1950, S. 440-455, 448. Es sind eine Reihe von speziellen Ausgestaltungen dieser Verfahren zur Herstellung von Alkalialkoholaten durch Umsetzung von Alkalimetall oder Alkalimetallamalgam mit Alkohol bekannt. Beispielsweise können diese Reaktionen durch Verwendung von Katalysatoren beschleunigt werden, wie in WO 99/65 849 A1, EP 1 195 369 A1 oder EP 153 3291 A1 beschrieben. Verfahren zur Reinigung des Rohprodukts sind ebenfalls bekannt, etwa die in WO 2004/048 624 A1 und WO 2004/048 625 A1 beschriebenen Verfahren zur Quecksilberentternung.

Allen Verfahren zur Herstellung von Alkalialkoholaten durch Umsetzung von Alkalimetall mit Alkohol ist gemeinsam, dass unumgesetzter oder überschüssiger Alkohol zunächst als sogenannter "Kristallalkohol" in einer Menge von ein bis drei Mol pro Mol Alkoholat im Rohprodukt verbleibt, was für viele Anwendungen von Alkoholaten, in denen protische Verbindungen wie Alkohole stören, unerwünscht ist.

Es sind verschiedene Verfahren bekannt, diesen Kristallalkohol zu vermeiden oder zu verringern. Beispielsweise wird in manchen Verfahren mit einem Unterschuss an Alkohol oder stöchiometrischem Einsatz des Alkohols, mit einem inerten Lösungs- oder Suspensionsmittel oder auch ohne solches gearbeitet, was die aufwendige Handhabung hochreaktiver unumgesetzter Alkalireste erfordert und deshalb sehr unbefriedigend ist. Üblicherweise wird daher mit mindestens stöchiometrischem Einsatz von Alkohol gearbeitet. Die im Labor übliche Umsetzung stöchiometrischer Mengen Alkohol und Alkali in einem inerten Lösungs- oder Suspensionsmittel ist technisch ebenfalls unbefriedigend. Einerseits ist dabei zusätzlicher Aufwand zur Abtrennung und Rückführung des inerten Lösungsmittels erforderlich, zudem neigt Alkalialkoxid in dieser Umgebung zur Bildung von Krusten auf dem Alkalimetall, so dass der Alkohol leicht mit diesem Alkoxid kristallalkoholhaltiges Alkoholat bildet und unumgesetztes Alkali zurückbleibt. Dieses Verfahren fordert also außerordentlich hohen Eintrag von mechanischer Energie zur steten Dispergierung von Alkali und Alkoholat einschließlich Abscheren der Alkoholatkrusten, was um so schwieriger und unwirtschaftlicher wird, je größer der verwendete Apparat ist. DD 298 502 A5 offenbart ein alternatives Verfahren, bei dem die Umsetzung von Alkali und Alkohol in einem siedenden inerten Lösungsmittel erfolgt, dessen Siedetemperatur oberhalb der Temperatur liegt, bei der Kristallalkohol aus dem Alkoholat entweicht. Dies ist ebenfalls unbefriedigend, da einerseits stets nur relativ geringe Mengen Alkohol dampfförmig im Reaktor vorliegen und so die Raumzeitausbeute gesenkt wird und andererseits der größtmögliche Aufwand bei Kondensation und Rückführung von Alkohol und auch Lösungsmittel erforderlich ist.

Üblicherweise wird deshalb Alkoholat in Form einer Lösung im entsprechenden Alkohol hergestellt und anschließend vom Alkohol befreit. Dazu wird das Lösungsmittel eingeengt, wobei kristallalkoholhaltiges Alkoholat aus der Lösung in kristalliner Form ausfällt (auskristallisiert). Durch Anwenden einer ausreichend hohen Temperatur und wahlweise auch Anlegen eines Vakuums kann auch der Kristallalkohol entfernt werden ("kalzinieren"). Das feste Produkt muss dabei kontinuierlich bewegt werden, um eine einheitliche Freiheit von Kristallalkohol zu erreichen und Verklumpungen zu verhindern.

Verfahren und Apparate zur Kristallisation einschließlich solcher zur Kristallisation durch Einengen eines Lösungsmittels sind seit langem allgemein bekannt. Ullmann's Encyclopedia of Industrial Chemistry, Sixth Edition, Wiley-VCH, Weinheim (ISBN 3-527-30385-5), gibt unter dem Stichwort "Crystallization and Precipitation" (Band 10, erschienen 2003) einen allgemeinen Überblick über derartige Verfahren. A.S. Myerson (Hrsg.): "Handbook of Industrial Crystallization", 2. Auflage, Butterworth-Heinemann, Boston 2002 (ISBN 0-7506-7012-6) und A. Mersmann (Hrsg.): Crystallization Technology Handbook", 2. Auflage, Dekker, New York 2001 (ISBN 0-8247-0528-9) geben einen Überblick über das Wissen des Fachmanns auf diesem Gebiet.

Die oben erwähnte WO 99/65 849 A1 und auch CN 1 239 714 A1 beschreiben derartige Verfahren in Schaufeltrocknern. US 3 587 704 offenbart ein aufwendiges Verfahren zur Gewinnung von pulverförmigem Natriumalkoxid aus Methanol in einem Dünnschichtverdampfer mit mechanischer Verteilung der einzudampfenden Lösung und nachgeschaltetem Zyklon. Das Produkt derartiger Verfahren hat den Nachteil, dass ein sehr hoher Feinkornanteil enthalten ist (Partikel mit unter 10 Mikrometer Durchmesser), d.h. das Alkalialkoxid stark staubt. Dies liegt einerseits an der mechanischen Belastung beim Kalzinieren und andererseits daran, dass durch das Entweichen des Kristallalkohols aus dem festen, kristallin vorliegenden Alkoholat die Gitterstruktur der Kristalle durch Effekte wie Kanalbildung bis hin zu Sprengung der Kristalle beeinträchtigt wird. Der hohe Feinkornanteil erhöht die Kohäsivität des Produkt-Haufwerks. Neben der so erschwerten Entleerung von Behältern sind auch entsprechende Schutzmaßnahmen gegen den stark ätzend wirkenden Staub erforderlich.

EP 1 306 125 A2 lehrt ein Verfahren zur Herstellung von Alkali- und Erdalkalialkoholat-Granulaten durch Wirbelschicht-Sprühgranulation von Alkoholatlösungen. Dabei ist die Fluidisierung des Produkts in der Wirbelschicht durch entsprechend große Gasströme notwendig. Zudem ist ein weiterer Schritt zur Abtrennung von Feinstaub samt Rückführung in die Granulation erforderlich. Beides ist wirtschaftlich nachteilig. Weiterhin ist bei den offenbarten Verfahrensbedingungen (z. B. 70-1335 °C) bei Normaldruck für die Granulation von Natriummethanolat) keinesfalls sicher, dass Kristallmethanol vollständig entfernt wird.

Es besteht weiterhin Bedarf an verbesserten Verfahren, insbesondere Verfahren zur einfachen und wirtschaftlichen Erzeugung von staubarmen und einfach handhabbaren Alkoholaten.

Dem gemäß wurde ein Verfahren zur Gewinnung von Alkoholaten durch Kristallisation aus einer Lösung im entsprechenden Alkohol gefunden, das dadurch gekennzeichnet ist, dass man das Alkoholat bei einem Druck von mindestens 3 bar abs. und einer Temperatur von mindestens 120 °C auskristallisiert.

Das erfindungsgemäße Verfahren führt zum Entstehen eines Produkts mit enger Partikelgrößenverteilung und sehr niedrigem Feinstaubanteil. Das Produkt ist gut handhabbar und neigt nicht zum Verbacken. Es sind gegenüber bekannten einfachen Verfahren keine zusätzlichen Apparate oder Verfahrensschritte erforderlich. Ein besonderer Vorteil des erfindungsgemäßen Verfahrens ist es, dass dabei direkt kristallalkoholfreies Alkoholat auskristallisiert, so dass ein nachfolgender Kalzinierschritt und die dabei auftretenden mechanischen Belastungen der Kristalle entfallen können.

Beim erfindungsgemäßen Verfahren wird das Alkoholat bei einem Druck von im Allgemeinen mindestens 3 bar abs. auskristallisiert, vorzugsweise bei einem Druck von mindestens 3,5 bar abs. und in besonders bevorzugter Weise bei einem Druck von mindestens 4 bar abs. Der im erfindungsgemäßen Verfahren anzuwendende Druck ist nach oben im Prinzip nicht begrenzt. Aus wirtschaftlichen Gründen, insbesondere den rapide steigenden Investitions- und Betriebskosten von für höhere Drücke ausgelegten Druckapparaten wird jedoch ein durch die wirtschaftlichen Rahmenbedingungen des Einzelfalls gegebene Obergrenze des Drucks gewählt. Ferner wird diese Obergrenze so gewählt, dass beim eingestellten Druck und der eingestellten Temperatur ein befriedigendes Ergebnis der Kristallisation erzielt wird. Diese von den stoffsystemspezifischen thermodynamischen Rahmenbedingungen (Abhängigkeit der Löslichkeit des Alkoholats von Temperatur und Druck) abhängigen Werte lassen sich für ein gegebenes Alkohol-/Alkoholat-Stoffsystem entweder Stoffdatensamlungen entnehmen oder durch wenige Routineversuche leicht ermitteln. Beispielsweise wird das Verfahren bei einem Druck von höchstens 325 bar abs., vorzugsweise von höchstens 100 bar abs. und in besonders bevorzugter Form von höchstens 15 bar abs., beispielsweise bei höchstens 10 bar abs. oder bei höchstens 6 bar abs. ausgeübt. Die vorstehenden Druckangaben sind Absolutwerte ("abs."), also keine "Überdruck"-Angaben ("barü"; barü = bar abs. - 1).

Beim erfindungsgemäßen Verfahren wird im Allgemeinen eine Temperatur von mindestens 120 °C eingestellt, vorzugsweise eine Temperatur von mindestens 125 °C und in besonders bevorzugter Form von mindestens 130 °C. Wie beim Druck wird die Obergrenze des einzustellenden Temperaturbereichs von wirtschaftlichen Rahmenbedingungen und von der Löslichkeit des Alkoholats im Alkohol bestimmt. Im Allgemeinen wird eine Temperatur von höchstens 250 °C, vorzugsweise von höchstens 200 °C und in besonders bevorzugter Weise von höchstens 180 °C eingestellt.

Mit dem erfindungsgemäßen Verfahren werden Alkoholate aus einer Lösung im entsprechenden Alkohol gewonnen. Insbesondere ist das Verfahren geeignet zur Gewinnung von Alkoholaten aliphatischer Alkohole, vor allem solchen mit einem geradkettigen oder verzweigten Kohlenstoffrest mit einem bis 8 Kohlenstoffatomen. In bevorzugter Weise werden primäre, sekundäre oder tertiäre Alkohole mit einem bis 5 Kohlenstoffatomen eingesetzt, und in besonders bevorzugter Weise solche mit einem bis vier Kohlenstoffatomen. Beispiele für im erfindungsgemäßen Verfahren eingesetzte Alkohole sind Methanol, Ethanol, n-Propanol, iso-Propanol, n-Butanol, 2-Butanol (sec.-Butanol) 2-Methyl-1-propanol (iso-Butanol), 2-Methyl-2-propanol (tert.-Butanol), 1-, 2- oder 3-Pentanol, neo-Pentanol, tert.-Pentanol, Hexan-, Heptan- oder Octanol. Ganz besonders bevorzugt ist Methanol. Gewonnen werden die entsprechenden Alkoholate.

Das Metall im Alkoholat, das mit dem erfindungsgemäßen Verfahren gewonnen wird, kann jedes Metall sein, das mit dem gewählten Alkohol ein Alkoholat bildet. Bevorzugt sind die Alkalimetalle Lithium, Natrium, Kalium, Rubidium und Cäsium sowie die Erdalkalimetalle Beryllium, Calcium, Magnesium, Strontium und Barium. Besonders bevorzugt sind die Alkalimetalle und ganz besonders bevorzugt ist Natrium.

Mit dem erfindungsgemäßen Verfahren werden folglich in besonders bevorzugter Weise Lithium-, Natrium- und Kalium- -methanolat, -ethanolat, -n-propanolat, -isopropanolat, -n-butanolat, -tert.-butanolat, -1-, 2- oder 3-peritanolat, -neo-pentanolat, tert.-pentanolat, -hexanolat, -heptanolat oder -octanolat (Trivialnamen jeweils "-y-" statt "-ano-") gewonnen und in ganz besonders bevorzugter Weise Natriummethanolat.

Zur Durchführung des erfindungsgemäßen Verfahrens wird zunächst auf bekannte Weise eine Lösung des zu gewinnenden Alkoholats im entsprechenden Alkohol erzeugt, beispielsweise durch Umsetzung des Metalls oder Metallamalgams mit dem Alkohol. Die Lösung kann, falls notwendig, vor dem Auskristallisieren des Alkoholats auf bekannte Weise von Verunreinigungen befreit werden, etwa bei Herstellung durch Umsetzung des Metallamalgams mit Alkohol durch die bekannten Destillations- oder Filtrationsverfahren von Quecksilber befreit werden, oder durch Dekantieren oder Filtrieren von unumgesetzten Verunreinigungen des Metalls.

Das Alkoholat wird in einem üblichen Apparat aus dieser Lösung durch Einengen auskristallisiert. Es kann jeder zum Auskristallisieren durch Einengen geeignete Apparat verwendet werden, der für den erfindungsgemäß anzuwendenden Druck und für die erfindungsgemäß anzuwendende Temperatur ausgelegt ist. Geeignet zur Durchführung des erfindungsgemäßen Verfahrens sind beispielsweise Schaufeltrockner, Rührkessel oder die so genannten Zwangsumlauf-, Leitrohr- oder Oslo-Kristallisatoren. Bevorzugterweise wird das erfindungsgemäße Verfahren in einem Schaufeltrockner durchgeführt.

Es ist möglich, das Alkoholat aus der Lösung fraktioniert auszukristallisieren, dies ist jedoch im Allgemeinen nur dann notwendig, wenn die zur Herstellung des Alkoholats verwendeten Einsatzstoffe unüblich stark verunreinigt sind.

Kristallisationsverfahren und -parameter sind, soweit hier nicht explizit ausgeführt, ebenso wie Kristallisatoren bekannt, s. z. B. die oben genannten Übersichtswerke zur Kristallisation, auf die hiermit ausdrücklich Bezug genommen wird, sowie den genannten Stand der Technik zur vorbekannten Verfahren zur Gewinnung von Alkoholaten, auf den ebenso ausdrücklich Bezug genommen wird.

### Beispiele

### Vergleichsbeispiel 1

In einem Schaufeltrockner mit 300 l Volumen wurde innerhalb von 4 Stunden 30 Gew.-%-ige Natriummethanolatlösung in Methanol bei Normaldruck durch Erwärmen knapp oberhalb des Siedepunkts von Methanol eingedampft. Nach vollständiger Entfernung des Lösungsmittels wurde die Temperatur auf 150 °C erhöht, um aus dem Natriummethanolat den Kristallmethanol auszutreiben. Der dadurch erhaltene Feststoff wies eine mittlere Partikelgröße von 95 Mikrometer auf, 9 Gew.-% des Feststoffs waren Partikel mit einem Durchmesser von weniger als 10 Mikrometer. Das Produkt staubte stark (Staubzahl von rund 110) und neigte auch in kleineren Gebinden zum Verbacken. (Verfahren zur Bestimmung der Staubzahl sind bekannt, s. z. B. die Übersicht von F. Hamelmann und E. Schmidt, Chemie Ingenieur Technik 74 (12) (2002) 1666-1676.)

### Beispiel 1

Vergleichsbeispiel 1 wurde wiederholt, wobei jedoch ein Druck von 4,5 bar abs. und eine Temperatur von konstant 140 °C eingestellt wurde. Der nach vollständiger Entfernung des Methanols erhaltene Feststoff war kristallmethanolfreies Natriummethanolat, wies eine mittlere Partikelgröße von 185 Mikrometer auf und nur 2 Gew.-% davon waren Partikel mit einem Durchmesser von weniger als 10 Mikrometer. Das Produkt staubte erheblich weniger als das Produkt von Vergleichsbeispiel 1 (Staubzahl 42) und blieb in Gebinden frei rieselfähig.

## Patentansprüche

1. Verfahren zur Gewinnung von Alkoholaten durch Kristallisation aus einer Lösung im entsprechenden Alkohol, **dadurch gekennzeichnet, dass** man das Alkoholat bei einem Druck von mindestens 3 bar abs. und einer Temperatur von mindestens 120 °C auskristallisiert.

2. Verfahren nach Anspruch 1, **dadurch gekennzeichnet, dass** man einen Druck von mindestens 3,5 bar abs. anwendet.

3. Verfahren nach Anspruch 2, **dadurch gekennzeichnet, dass** man einen Druck von mindestens 4 bar abs. anwendet.

4. Verfahren nach Anspruch 1, **dadurch gekennzeichnet, dass** man eine Temperatur von mindestens 125 °C anwendet.

5. Verfahren nach Anspruch 4, **dadurch gekennzeichnet, dass** man eine Temperatur von mindestens 130 °C anwendet.

6. Verfahren nach Anspruch 1, **dadurch gekennzeichnet, dass** man einen Druck von mindestens 4 bar abs. und eine Temperatur von mindestens 130 °C anwendet.

7. Verfahren nach Ansprüchen 1 oder 6, **dadurch gekennzeichnet, dass** man Alkalialkoholat gewinnt.

8. Verfahren nach Anspruch 7, **dadurch gekennzeichnet, dass** man Natriumalkoholat gewinnt.

9. Verfahren nach Anspruch 8, **dadurch gekennzeichnet, dass** man Natriummethanolat gewinnt.

## Claims

1. A method of isolating alkoxides by crystallization from a solution in the corresponding alcohol, wherein the alkoxide is crystallized out at a pressure of at least 3 bar abs. and a temperature of at least 120°C.

2. The method according to claim 1, wherein a pressure of at least 3.5 bar abs. is employed.

3. The method according to claim 2, wherein a pressure of at least 4 bar abs. is employed.

4. The method according to claim 1, wherein a temperature of at least 125°C is employed.

5. The method according to claim 4, wherein a temperature of at least 130°C is employed.

6. The method according to claim 1, wherein a pressure of at least 4 bar abs. and a temperature of at least 130°C are employed.

7. The method according to claim 1 or 6, wherein an alkali metal alkoxide is isolated.

8. The method according to claim 7, wherein a sodium alkoxide is isolated.

9. The method according to claim 8, wherein sodium methoxide is isolated.

## Revendications

1. Procédé de production d'alcoolates par cristallisation à partir d'une solution dans l'alcool correspondant, **caractérisé en ce qu'**on sépare l'alcoolate par cristallisation à une pression d'au moins 3 bars abs. et à une température d'au moins 120°C.

2. Procédé selon la revendication 1, **caractérisé en ce qu'**on utilise une pression d'au moins 3,5 bars abs.

3. Procédé selon la revendication 2, **caractérisé en ce qu'**on utilise une pression d'au moins 4 bars abs.

4. Procédé selon la revendication 1, **caractérisé en ce qu'**on utilise une température d'au moins 125°C.

5. Procédé selon la revendication 4, **caractérisé en ce qu'**on utilise une température d'au moins 130°C.

6. Procédé selon la revendication 1, **caractérisé en ce qu'**on utilise une pression d'au moins 4 bars abs. et une température d'au moins 130°C.

7. Procédé selon les revendications 1 ou 6, **caractérisé en ce qu'**on obtient un alcoolate de métal alcalin.

8. Procédé selon la revendication 7, **caractérisé en ce qu'**on obtient un alcoolate de sodium.

9. Procédé selon la revendication 8, **caractérisé en ce qu'**on obtient du méthanolate de sodium.
